# EUROPEAN PATENT SPECIFICATION

(11) **EP 1 726 579 B1**
(45) Date of publication and mention of the grant of the patent: **01.09.2010**
(21) Application number: 04788221.2
(22) Date of filing: 28.09.2004
(51) Int. Cl.: C07C 67/08

(54) **PROCESS FOR PRODUCING (METH)ACRYLIC ESTER**
VERFAHREN ZUR HERSTELLUNG VON (METH)ACRYLSÄUREESTER
PROCEDE DE PRODUCTION D'ESTER (METHA)CRYLIQUE

(30) Priority: 19.03.2004 JP 2004080725
(43) Date of publication of application: 29.11.2006
(73) Proprietor: Mitsubishi Chemical Corporation, Minato-ku Tokyo 108-0014 (JP)
(72) Inventor: YADA, Shuhei, c/o Mitsubishi Chemical Corp., Mie 5108530 (JP); TAKASAKI, Kenji, c/o Mitsubishi Chemical Corp., Mie 5108530 (JP); SUZUKI, Yoshiro, c/o Mitsubishi Chemical Corp., Mie 5108530 (JP); OGAWA, Yasushi, c/o Mitsubishi Chemical Corp., Mie 5108530 (JP)
(74) Representative: Woods, Geoffrey Corlett
(86) International application number: PCT/JP2004/014154
(87) International publication number: WO 2005/090279

(56) References cited:
- JP-A- 3 240 753
- US-A- 4 733 004
- US-A1- 2002 189 927

## Description

### TECHNICAL FIELD

The present invention relates to a process for producing (meth)acrylic esters. Meanwhile, in the present specification, the "(meth)acrylic esters" generally include acrylic esters and methacrylic esters.

### BACKGROUND ARTS

As catalysts for production of (meth)acrylic esters by esterifying (meth)acrylic acid with a C₁ to C₄ alcohol, there have been extensively used strong acid cation exchange resins. The catalysts for the esterification reaction process are generally used in the form of a fixed bed, but may also be used in the form of a fluidized bed. Further, since the esterification reaction is an equilibrium reaction, in the industrial production of (meth)acrylic esters, there has been adopted such a recovery step in which unreacted raw materials are separated from the reaction solution by distillation and recycled to the esterification reaction step (for example, Japanese Patent Publication (KOKOKU) Nos. 6-86405 and 6-86406).

### DISCLOSURE OF THE INVENTION

### PROBLEM TO BE SOLVED BY THE INVENTION

Meanwhile, in the industrial production of (meth)acrylic esters, it is an important task to prolong a life of catalysts used therein.

The present invention has been made for solving these conventional problems. An object of the present invention is to provide an industrially advantageous process for producing (meth)acrylic esters which is improved so as to prolong a life of a strong acid cation exchange resin catalyst used therein.

### MEANS FOR SOLVING THE PROBLEM

As a result of the present inventors' earnest studies, it has been found that when a trace amount of solids such as polymeric heavy components and finely crushed resins contained in the reaction solution are removed therefrom, a life of the strong acid cation exchange resin catalyst used therein can be unexpectedly prolonged.

Meanwhile, it is considered that the above polymeric heavy components and finely crushed resins are produced as follows. That is, nevertheless adding a polymerization inhibitor to (meth)acrylic acid upon handling, a trace amount of polymeric heavy components are irreversibly produced from the (meth)acrylic esters by heat generated at distillation columns used in the reaction step and recovery step. Further, the strong acid cation exchange resin catalyst used in the production process tends to suffer from oxidation deterioration with the passage of time as well as repeated swelling and shrinkage, resulting in occurrence of cracks therein and, therefore, production of finely crushed resins.

Further, it is considered that the shortened life of the strong acid cation exchange resin catalyst due to formation of the solids is caused as follows. That is, active sites of the catalyst are covered with the polymeric heavy components as produced, resulting in the shortened life of the catalyst. In particular, in the case of fixed bed type catalysts, results of the reaction tend to be deteriorated owing to deviated flow which is attributed to clogging by polymeric heavy components and finely crushed resins.

The present invention has been attained on the basis of the above finding. To accomplish the aim, in an aspect of the present invention, there is provided a process for producing a (meth)acrylic ester, comprising:
a reaction step of esterifying (meth)acrylic acid with a C₁ to C₄ alcohol in the presence of a strong acid cation exchange resin catalyst to produce the (meth)acrylic ester;
a recovery step of separating an unreacted (meth)acrylic acid from a reaction solution obtained in the reaction step; and
a recycling step of recycling the thus recovered unreacted (meth)acrylic acid to the reaction step,
solids, including finaly crushed resins of the catalyst, contained in the recovered unreacted (meth)acrylic acid to be recycled to the reaction step being separated therefrom.

### EFFECT OF THE INVENTION

According to the present invention, there is provided a process for producing (meth)acrylic esters which is capable of prolonging a life of a strong acid cation exchange resin catalyst used therein and ensuring a stable continuous operation thereof for a long period of time.

### BRIEF DESCRIPTION OF THE DRAWING

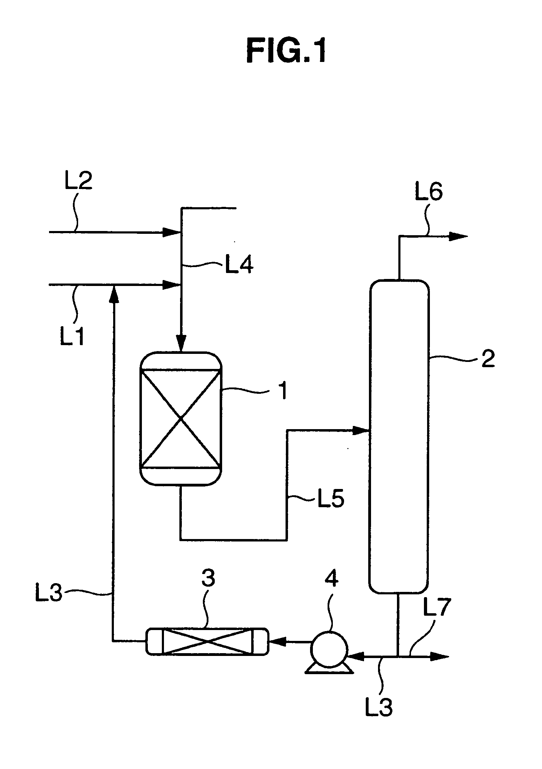

Fig. 1 is an explanatory view showing an essential part of a preferred embodiment of the production process according to the present invention.

### EXPLANATION OF REFERENCE NUMERALS

1: Reactor; 2: Distillation column; 3: Solid separation means

### PREFERRED EMBODIMENT FOR CARRYING OUT THE INVENTION

The present invention is described in detail below.
The alcohol used as a raw material in the production process of the present invention is a C₁ to C₄ alcohol. Specific examples of the alcohol may include methanol, ethanol, i-propanol, n-propanol, n-butanol, i-butanol, sec-butanol and t-butanol. In addition, unreacted alcohol separated and recovered in the below-mentioned recovery step may be recycled and reused as the raw alcohol. Specific examples of the (meth)acrylic esters obtained by the production process of the present invention may include methyl (meth)acrylate, ethyl (meth)acrylate, isopropyl (meth)acrylate and butyl (meth)acrylate. Among these (meth)acrylic esters, especially preferred are methyl acrylate, ethyl acrylate and methyl methacrylate.

The strong acid cation exchange resin used as the catalyst may be of either a porous type or a gel type. The strong acid cation exchange resin has a crosslinking degree of usually 2 to 16%. Examples of the commercially available strong acid cation exchange resin may include porous type strong acid cation exchange resins "PK-208", "PK-216" and "PK-228" all produced by Mitsubishi Chemical Corporation or the like.

In Fig. 1, there is shown an essential part of a preferred embodiment of the production process according to the present invention. The production process of the present invention comprises a reaction step of esterifying (meth)acrylic acid with a C₁ to C₄ alcohol in the presence of a strong acid cation exchange resin catalyst to produce a (meth)acrylic ester; a recovery step of separating an unreacted (meth)acrylic acid from a reaction solution obtained in the reaction step; and a recycling step of recycling the recovered unreacted (meth)acrylic acid to the reaction step, similarly to the conventionally known processes.

More specifically, in the reaction step, a reactor (1) packed with the strong acid cation exchange resin catalyst, is charged with (meth)acrylic acid through a line (L1) and with alcohol through a line (L2). Reference numeral (L3) denotes a circulation line for the recovered (meth)acrylic acid as mentioned below, and reference numeral (L4) denotes a circulation line for the recovered alcohol as mentioned below.

The reaction conditions may be appropriately selected according to the raw materials used. The molar ratio of alcohol to (meth)acrylic acid is usually 0.5 to 2.0. The reaction temperature is usually 50 to 90°C, and the reaction time (residence time) is usually 1 to 5 hours. In the esterification reaction, water is by-produced together with the aimed (meth)acrylic esters. Therefore, the reaction solution is a mixture of these products and the unreacted (meth)acrylic acid and alcohol. Meanwhile, the reaction step as illustrated in Fig. 1 is conducted in a fixed bed of a continuous type. However, the reaction step may also be conducted in a fluidized bed type or a batch type.

More specifically, in the recovery step, the reaction solution is withdrawn from the reactor (1) through a line (L5), and fed to a distillation column (2) for treatment thereof. In most cases where the C₁ to C₄ alcohol is used in the production process, an azeotropic mixture formed therein may be any of water/alcohol, water/(meth)acrylic ester, alcohol/(meth)acrylic ester, and water/alcohol/(meth)acrylic ester. For this reason, the (meth)acrylic acid may be frequently separated from the other three components by azeotropy of these mixtures or difference in boiling point from that of the (meth)acrylic ester.

Examples of the distillation column may include perforated plate columns, bubble-cap columns, packed columns and combinations of these columns (for example, combination of the perforated plate column and the packed column). Any of these distillation columns may also be used irrespective of provision of overflow weirs or down corners.

Examples of the trays may include trays having a downcomer such as a bubble-cap tray, a perforate plate tray, a bubble tray, a super-flux tray and a max-flux tray, and trays having no downcomer such as a dual flow tray.

Examples of packing materials used in the distillation column may include conventional packing materials having various shapes such as a solid cylindrical shape, a hollow cylindrical shape, a saddle shape, a spherical shape, a cubic shape and a pyramidal shape as well as commercially available regular or irregular packing materials having specific shapes which have been recently noticed as high-performance packing materials.

Examples of the commercially available regular packing materials may include gauze-type regular packing materials such as "SULZER PACKING" produced by Sulzer Brothers Limited, "SUMITOMO SULZER PACKING" produced by Sumitomo Heavy Industries Ltd., "TECHNO PACK" produced by Mitsui & Co., Ltd., sheet-type regular packing materials such as "MELLAPAK" produced by Sumitomo Heavy Industries Ltd., "MC PACK" produced Mitsubishi Chemical Engineering Corporation, and grid-type regular packing materials such as "FLEXI-GRID" produced by Cork Co., Ltd.; as well as "GEM-PAK" produced by Grich Inc., "MONTZPACK" produced by Montz Inc., "GOODROLL PACKING" produced by Tokyo Special Wire Netting Co. Ltd., "HONEYCOMB PACK" produced NGK INSULATORS, LTD., and "IMPULSE PACKING" produced NAGAOKA Corporation.

Examples of the commercially available irregular packing materials may include Rashig ring, "Pole Rings" both produced by BASF AG, "Cascade Mini-Ring" produced by Mass-Transfer Inc., "IMTP" and "INTERLOCKS SADDLE" both produced by Norton Inc., "TELLERETT" produced by Nittetu Chemical Engineering Ltd., and "FLEXI RINGS" produced by JGC CORPORATION

These packing materials may be used in the combination of any two or more thereof, and may also be used in combination with conventionally used trays.

The operation conditions of the above distillation column may vary depending upon compositions of raw materials to be distilled, recovery rate, etc. However, since the (meth)acrylic ester is an easily-polymerizable compound, the distillation temperature and pressure are preferably set as low as possible. More specifically, the temperature at a bottom of the distillation column is usually in the range of 60 to 100°C, and the pressure at a top of the distillation column is usually in the range of 1.33 to 26.7 kPa. Meanwhile, top components recovered from a top of the distillation column such as (meth)acrylic esters, water and unreacted alcohol are discharged through a line (L6) and fed to the other distillation column to recover the unreacted alcohol therefrom.

Meanwhile, upon the reaction and distillation, in order to suppress production loss due to undesired polymerization of (meth)acrylic acid or corresponding esters thereof, a polymerization inhibitor or a polymerization preventive agent such as oxygen-containing gases may be added to the reactors or the distillation columns.

The means for separating the unreacted (meth)acrylic acid from the reaction solution is not limited to the distillation means, and there may also be used an extraction means using various extractants such as alkalis as well as the combination of the distillation means and the extraction means.

The present invention is **characterized in that** solids contained in the unreacted (meth)acrylic acid to be recycled to the reaction step are separated therefrom. In the embodiment illustrated in Fig. 1, a solid separation means (3) is provided in the course of the circulation line (L3) for the recovered (meth)acrylic acid. Meanwhile, reference numeral (4) denotes a pump provided in the course of the circulation line (L3) for recycling, and reference numeral (7) denotes a discharge line for discharging a bottom liquid from the distillation column (2).

The solid separation means (3) is not limited to specific types as far as a trace amount of solids contained in the unreacted (meth)acrylic acid to be recycled can be separated therefrom. In the consideration of operability and costs required, as the solid separation means, there may be used a filtering means such as strainers, filters and centrifugal filters. In particular, of these filter means, most preferred are filters of a cartridge type because of continuous operability, low installation costs and good operating property. The diameter of particles penetrated through the filters is preferably in the range of 1 to 10 µm because almost all diameters of solid particles to be separated are involved within the above-specified range. As the material of the filters, there may be used glass fibers, polyesters, Teflon (registered trademark), polypropylene, polyamides or the like. Of these materials, polypropylene is preferred from the standpoints of high strength, good acid resistance and low costs.

The solids to be separated are polymeric heavy components or finely crushed resins as described above. Examples of the polymeric heavy components may include poly(meth)acrylic acid, poly(meth)acrylic esters and Michael adduct-type high-molecular polyesters. Also, the solids include sludge such as iron rusts. The temperature used upon separating the solids is usually 50 to 90°C from the standpoint of maintaining a solid state of the above polymeric heavy components.

### EXAMPLES

The present invention is described in more detail below by Examples, but the Examples are only illustrative and not intended to limit the scope of the present invention.

### Example 1:

According to the process shown in Fig. 1, methyl acrylate was continuously produced. A strong acid cation exchange resin "PK-216" produced by Mitsubishi Chemical Corporation was used as a catalyst in the form of a fixed bed in the production process. Also, a cartridge filter capable of passing particles having a diameter of 3 µm or lower therethrough was used as the solid separation means (3).

The reactor packed with the catalyst was charged with acrylic acid, methanol, recovered acrylic acid and recovered methanol through the line (L1), the line (L2), the circulation line (L3) and the circulation line (L4), respectively. The flow rates of the fresh raw materials and the recovered raw materials were controlled such that the molar ratio of acrylic acid to methanol fed was about 1.25:1, and the reaction temperature was kept constant at about 75°C.

It was confirmed that the reaction mixture obtained at an outlet of the esterification reactor was composed of 12.2% by weight of water, 4.1% by weight of methanol, 38.4% by weight of methyl acrylate, 23.3% by weight of acrylic acid, and 22.0% by weight of others, and an average flow rate thereof was 7.5 tons/h and, therefore, was kept substantially unchanged.

On the other hand, the reaction solution was withdrawn from the reactor (1), and then treated in the distillation column (2). At this time, a methanol solution containing 5% by weight of hydroquinone (polymerization inhibitor) was fed to the distillation column (2) at a feed rate of 35.4 Kg/h. The top pressure of the distillation column was 26 kPa, the reflux ratio was 1, and the bottom temperature was 80°C on the average. Further, the top components obtained from the top of the distillation column were composed of 16% by weight of water, 8.3% by weight of methanol, 70.2% by weight of methyl acrylate and 5.2% by weight of others, and an average flow rate thereof was 4.1 tons/h and, therefore, was kept substantially unchanged. In addition, the bottom components obtained from the bottom of the distillation column were composed of 7.5% by weight of water, 0.1% by weight of methyl acrylate, 50.5% by weight of acrylic acid and 41.8% by weight of others, and an average flow rate thereof was 3.4 tons/h and, therefore, was kept substantially unchanged.

The above bottom components were recycled through the circulation line (L3) to the above reactor (1). At this time, the temperature of the solid separation means (3) (cartridge filter) was kept constant at about 80°C. Meanwhile, using another distillation column, unreacted methanol was separated and recovered from the above top components, and then recycled to the reactor (1).

The above continuous operation was performed for 2 years. As a result, it was confirmed that after the elapse of 2 years, the conversion rate of acrylic acid at the outlet of the reactor (1) was lowered by only less than 1% on the average as compared to that upon initiation of the operation.

### Comparative Example 1:

The same procedure as defined in Example 1 was conducted except that no filter was provided, thereby performing the continuous operation. As a result, it was confirmed that after the elapse of 1.1 months, the conversion rate of acrylic acid was lowered by 3.7% as compared to that upon initiation of the operation. Thus, since the performance of the purification system approached its limit, the catalyst used therein was replaced with a new one upon the periodical repair.

## Claims

1. A process for producing a (meth)acrylic ester, comprising:
a reaction step of esterifying (meth)acrylic acid with a C₁ to C₄ alcohol in the presence of a strong acid cation exchange resin catalyst to produce the (meth)acrylic ester;
a recovery step of separating an unreacted (meth)acrylic acid from a reaction solution obtained in the reaction step; and
a recycling step of recycling the thus recovered unreacted (meth)acrylic acid to the reaction step,
solids, including finely crushed resins of the catalyst, contained in the recovered unreacted (meth)acrylic acid to be recycled to the reaction step being separated therefrom.

2. A process according to claim 1, wherein a means for separating the solids is a filter.

3. A process according to claim 2, wherein the diameter of particles penetrated through said filter is in the range of 1 to 10 µm.

4. A process according to any one of claims to 3, wherein the solids are separated from the recovered unreacted (meth)acrylic acid at a temperature 50 to 90°C.

5. A process according to any one of claims 1 to 4, wherein the (meth)acrylic ester is methyl acrylate, ethyl acrylate or methyl methacrylate.

## Patentansprüche

1. Verfahren zur Herstellung eines (Meth)Acrylesters, umfassend:
einen Reaktionsschritt des Veresterns von (Meth)Acrylsäure mit einem C₁- bis C₄-Alkohol in Gegenwart eines starken sauren Kationentauscherharzkatalysators unter Herstellung des (Meth)Acrylesters;
einen Gewinnungsschritt des Trennens von nichtumgesetzter (Meth)Acrylsäure von einer im Reaktionsschritt erhaltenen Reaktionslösung;
und einen Recyclingschritt des Recycelns der so gewonnenen nichtumgesetzten (Meth)Acrylsäure zu dem Reaktionsschritt, wobei
Feststoffe, einschließlich fein zerkleinerter Harze des Katalysators, enthalten in der gewonnenen nichtumgesetzten (Meth)Acrylsäure, die zu dem Reaktionsschritt recycelt werden soll, davon abgetrennt werden.

2. Verfahren gemäß Anspruch 1, wobei ein Mittel zum Abtrennen der Feststoffe ein Filter ist.

3. Verfahren gemäß Anspruch 2, wobei der Durchmesser der Partikel, die durch den Filter dringen, in dem Bereich von 1 bis 10 µm liegt.

4. Verfahren gemäß einem beliebigen der Ansprüche 1 bis 3, wobei die Feststoffe von der gewonnenen nichtumgesetzten (Meth)Acrylsäure bei einer Temperatur von 50 bis 90°C abgetrennt werden.

5. Verfahren gemäß einem beliebigen der Ansprüche 1 bis 4, wobei der (Meth)Acrylester Methylacrylat, Ethylacrylat oder Methylmethacrylat ist.

## Revendications

1. Procédé de production d'un ester (méth)acrylique, comprenant :
une étape réactionnelle consistant à estérifier l'acide (méth)acrylique avec un alcool de C₁ à C₄ en présence d'un catalyseur en résine à échange cationique d'acide fort, dans le but de produire l'ester (méth)acrylique ;
une étape de récupération consistant à séparer l'acide (méth)acrylique n'ayant pas réagi d'une solution réactionnelle obtenue à l'étape réactionnelle ; et
une étape de recyclage consistant à recycler l'acide (méth)acrylique n'ayant pas réagi et récupéré de la sorte à l'étape réactionnelle ;
la séparation des solides, y compris des résines finement broyées du catalyseur, contenus dans l'acide (méth)acrylique n'ayant pas réagi et récupéré à l'étape réactionnelle pour être recyclé, de ce dernier.

2. Procédé selon la revendication 1, dans lequel un moyen permettant de séparer les solides est un filtre.

3. Procédé selon la revendication 2, dans lequel le diamètre des particules qui pénètrent à travers ledit filtre se situe dans la plage de 1 à 10 µm.

4. Procédé selon l'une quelconque des revendications 1 à 3, dans lequel les solides sont séparés de l'acide (méth)acrylique n'ayant pas réagi et récupéré, à une température de 50 à 90 °C.

5. Procédé selon l'une quelconque des revendications 1 à 4, dans lequel l'ester d'acide (méth)acrylique est l'acrylate de méthyle, l'acrylate d'éthyle ou le méthacrylate de méthyle.
